# EUROPÄISCHE PATENTSCHRIFT

(11) **EP 4 114 283 B1**
(45) Veröffentlichungstag und Bekanntmachung des Hinweises auf die Patenterteilung: **11.06.2025**
(21) Anmeldenummer: 21709650.2
(22) Anmeldetag: 02.03.2021
(51) Int. Cl.: A61B 17/132, A61B 90/92, A61B 17/12, A61B 17/00, A61B 90/00

(54) **ABDRÜCKHILFE**
TOURNIQUET
TOURNIQUET

(30) Priorität: 05.03.2020 DE 102020105891
(43) Veröffentlichungstag der Anmeldung: 11.01.2023
(73) Patentinhaber: Fresenius Medical Care Deutschland GmbH, 61352 Bad Homburg (DE)
(72) Erfinder: MARTERSTOCK, Stefan Konrad, 97337 Dettelbach (DE); KOPPERSCHMIDT, Pascal, 97456 Dittelbrunn (DE); SPICKERMANN, Reiner, 97535 Wasserlosen-Burghausen (DE)
(74) Vertreter: Herrmann, Uwe
(86) Internationale Anmeldenummer: PCT/EP2021/055096
(87) Internationale Veröffentlichungsnummer: WO 2021/175804

(56) Entgegenhaltungen:
- WO-A1-2019/017828
- CN-A- 107 714 132
- DE-A1- 102013 112 597
- US-A1- 2006 201 522
- US-A1- 2014 135 819
- US-A1- 2018 187 703
- US-A1- 2019 247 054

## Beschreibung

Die vorliegende Erfindung betrifft eine Abdrückhilfe zum Abdrücken einer Punktionsstelle, insbesondere eines venösen Zugangs eines Patienten.

Nach einer Hämodialysebehandlung ist der Patient meist heparinisiert, d.h. die Blutgerinnung ist noch negativ beeinträchtigt. Infolgedessen muss der venöse Zugang abgedrückt werden, nachdem die Nadel der Dialysemaschine diskonnektiert wurde.

Die aus dem Stand der Technik bekannten Abdrückhilfen basieren meist auf dem einfachen Federkraftmechanismus. Dabei kann über einen Drehknopf die Kraft auf die Punktionsstelle eingestellt werden. Dies wird mit einem Schraubenmechanismus realisiert (Kraft - Weg - Beziehung). Dabei ergibt sich der Nachteil, dass der ausgeübte Druck falsch eingestellt werden kann. Noch nachteiliger ist, dass man bei Bewegung/Muskelanspannung, d.h. Veränderung des Armumfangs, die ausgeübte Druckkraft beeinflusst, was in der Konsequenz ggf. zu einer Schädigung des venösen Zugangs führen kann und oder zumindest unangenehm hinsichtlich des Tragekomforts ist.

Abgesehen davon sind die bekannten Abdrückhilfen nicht flach und passen daher nur schlecht unter die Kleidung des Patienten.

Aus dem Stand der Technik ist es ebenfalls bekannt, einen Kompressor und ein Luftkissen zur dynamischen Druckausübung auf die Punktionsstelle zu nutzen. Auf diese Weise kann die ausgeübte Kraft bzw. der Druck immer im idealen Bereich gehalten werden. Die Realisierung ist jedoch vergleichsweise aufwendig (Medizinprodukte-Software und Hardware). Unter anderem ist ein Akkumulator nötig, um das Gerät zu betreiben. Die Verfügbarkeit ist also nur gewährleistet, wenn der Akku auch regelmäßig vollgeladen wird, usw. Zudem ist die Portabilität des Geräts ein weiterer Nachteil im Vergleich zu den mechanischen Lösungen.

Beispierlhafter Stand der Technik ist beispielsweise US2019/247054A1. CN107714132A offenbart ein Band zum Abdrücken eines Arms. US2014/135819A1 offenbart ein Band aus Neopren zum Abdrücken eines Arms oder Beins. Eine weitere Abdrückhilfe ist aus der DE102013112597A1 bekannt. WO2019/017828A1 offenbart eine medizinische Kompressionsvorrichtung. Eine weitere Abdrückhilfe offenbart die US2018/187703A1. US2006/201522A1 offenbart einen Kompressionsstreifen mit einem Anzeigeelement.

Der vorliegenden Erfindung liegt somit die Aufgabe zugrunde, eine Abdrückhilfe bereitzustellen, die zuverlässig zu bedienen ist und einen einfachen Aufbau aufweist.

Diese Aufgabe wird durch eine Abdrückhilfe mit den Merkmalen des Anspruchs 1 gelöst. Danach ist vorgesehen, dass die Abdrückhilfe ein umlaufendes Band umfasst, dessen Umfang verstellbar ist, wobei in dem umlaufenden Band eine Konstantkraftfeder angeordnet ist. Eine Konstantkraftfeder bringt den Vorteil mit sich, dass diese anders als eine konventionelle Feder über einen bestimmten Weg bzw. Hub eine konstante Kraft ausübt bzw. einregelt. Dies ermöglicht es, die Wahrscheinlichkeit dafür zu verringern, dass auf die Punktionsstelle eine zu geringe oder eine zu hohe Presskraft ausgeübt wird. Für den Nutzer genügt es, die Abdrückhilfe so einzustellen, dass der Hub bzw. die Auslenkung im Bereich der Konstantkraft liegt. Die Erfindung sieht somit eine Konstantkraftfeder vor, welche partiell eine Unabhängigkeit zwischen Kraft und Weg bietet. Mögliche Realisierungsformen sind rein mechanisch oder auch magnetisch.

Erfindungsgemäß ist vorgesehen, dass sich vorzugsweise auf der Außenseite der Konstantkraftfeder eine Anzeigeelement befindet, das ausgebildet ist, die Auslenkung der Konstantkraftfeder unmittelbar oder mittelbar anzuzeigen. Dies ermöglicht es dem Nutzer festzustellen, ob der Bereich der Konstantkraft bereits erreicht oder noch unterschritten oder gar überschritten ist. Intention der Anzeige ist es, dass man die Konstantkraftfeder mittig auslenken kann, so dass man einen möglichst großen Regelbereich erhält. Die von der Feder aufgebrachte Kraft kann darüber nicht unmittelbar beeinflusst werden.

Vorteil der Anzeige ist die Optimierung des Aktionsbereichs, in dem die Feder die Kraft konstant halten kann.

Erfindungsgemäß ist das Anzeigeelement ausgebildet, um dem Nutzer den Bereich der Konstantkraft anzuzeigen.

Denkbar ist es, dass ein Element vorgesehen ist, mittels dessen die ausgeübte Kraft über den Bereich der durch die Konstantkraftfeder aufgebrachten Konstantkraft erhöht werden kann. Dieses Element dient dazu, im Fall eines zu starken Anziehens außerhalb der Idealposition (Mitte des Konstantkraftbereiches) oder im Notfall die Punktionsstelle mittels Verschluss fester abzudrücken, um eine Blu-tung stoppen zu können. Das Element kann hierfür bspw. partiell bis zu einem Anschlag über eine konventionelle Federkonstante verfügen (Elastisches Modul) oder als eine Art "Sicherungskette" ausgeführt sein.

Besonders im Notfall kann es notwendig sein, eine Kraft auf die Punktionsstelle auszuüben, die über der ideal aubringbaren Kraft der Kosntantfeder liegt. Diese für den Notfall vorgesehene Abdrückung sollte jedoch auch begrenzt oder auch unbegrenzt sein (Anschlag). Im Notfall kann nach eigenem Ermessen abgedrückt werden.

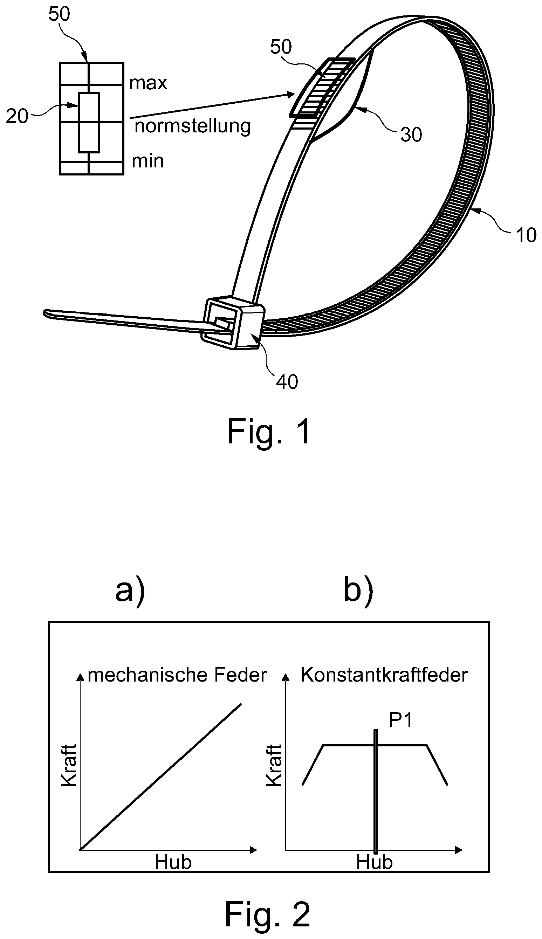

Hierfür kommen zwei (nicht beschränkende) exemplarisch genannte Alternativen in Betracht, die jeweils paralell und losgelöst zur eigentlichen Abdrückfeder am Band angebracht ist/sind:
1) Ein Sicherungselement, wie zum Beispiel eine Sicherungskette, die sicherstellt, dass der "Anziehweg" einen krirtischen Punkt (max.) nicht überschreitet. Durch Begrenzen des Anziehwegs wird somit auch die ausgeübte Kraft begrenzt. Diese Begrenzung hat den Sinn, zu verhindern, dass bei zu starkem Anziehen die Kraft der Konstantkraftfeder nachlässt (vgl. Kennlinie in Figur 2). Sobald sich die Feder außerhalb des Bereiches zwischen Min. und Max. befindet, liefert die Feder ggf. praktisch keine Kraft mehr, d.h. die Abdrückhilfe könnte sich komplett lösen, d.h. in zwei Teile auseinander gehen.
2) Ausführung mit Feder. Hierfür kann optional eine weitere Skala vorgesehen sein, die die ausgeübte Kraft relativ zum E-Modul des verwendeten Materials anzeigt. Eine Feinjustierung im Endbereich der max. ausübbaren Kraft kann hierdurch realisert werden.

Die Federhärte kann relativ zu der Kennlinie der Kontraftkraftfeder, insbesondere unter Berücksichtigung des Punktes P1, d.h. der Idealposition, ausgelegt sein.

Es muss für die max. ausübbare Kraft sichergestellt sein, dass der Verschluss des Bands nicht durchrutscht bzw. sich nicht ungewollt öffnet.

Somit besteht eine denkbare Ausführung darin, dass es sich bei dem Element um eine Feder handelt, die vorzugsweise wenigstens bereichsweise eine lineare Abhängigkeit zwischen Hub und Kraft aufweist, d.h. wie eine konventionelle Feder.

Vorzugsweise ist das genannte Element zu der Konstantkraftfeder parallel geschaltet. Auch eine serielle Schaltung ist denkbar und von der Erfindung umfasst.

Eine serielle Schaltung ggf. in Kombination mit einem Anschlag ist sinnvoll, sodass bei Überdehung in den Anschlag der Konstantkraftfeder diese dann verweilt und dann die konventionelle, in Serie geschaltete Feder eine Kraft-Hub-Kennlinie oberhalb der Kraft der Kontanstkraftfeder liefert.

Das Band kann zwei Enden aufweisen, wobei das eine Ende des Bandes in das andere Ende des Bandes einsteckbar und dort arretierbar ist, wie dies von einem Kabelbinder bekannt ist. Auch andere Verschlüsse sind von der Erfindung mit umfasst.

Vorzugsweise ist vorgesehen, dass das Band einen Verschluss aufweist, der manuell lösbar ist (Reuse, Entfernen der Abdrückhilfe).

Die Konstantkraftfeder kann mechanisch und/oder magnetisch ausgeführt sein. Der Begriff "Feder" ist somit weit auszulegen und umfasst auch magnetische Ausführungen oder z.B. auch rein mechanische Varianten.

Denkbar ist es, dass die Abdrückhilfe eine farbliche Kodierung aufweist, die mit der durch die Konstantkraftfeder aufgebrachte Kraft korreliert ist. So wird dem Nutzer sofort erkenntlich gemacht, welche Konstantkraft mit welcher Abdrückhilfe realisiert werden kann. Dies senkt auch das Risiko einer falschen Anwendung durch Verwechslung der verfügbaren Abdrückhilfen, da ein Patient mit der Zeit seine Stärke, d.h. ggf. Farbe kennt und es für ihn ersichtlich ist, wenn er eine falsche Abdrückhilfe von dem Personal bekäme.

Die vorliegende Offenbarung betrifft auch ein Verfahren zum Abdrücken einer Punktionsstelle, wie z.B. einer Fistel oder eines sonstigen Blutgefäßes, insbesondere des oder der Zugänge eines Dialysepatienten.

Die Abdrückhilfe kommt zum Einsatz, wenn die Nadel oder Kanüle entfernt wurde, wie z.B. nach der Durchführung einer Dialysebehandlung.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine perspektivische Ansicht einer Abdrückhilfe gemäß der Erfindung und
- Figur 2:: Kraft - Weg Verläufe einer konventionellen Feder sowie einer Konstantkraftfeder.

Figur 1 zeigt in perspektivischer Ansicht eine Abdrückhilfe gemäß der Erfindung.

Die Abdrückhilfe besteht aus einem umlaufenden Band 10, in dem über einen Teilumfang hinweg eine Konstantkraftfeder 20 integriert ist. Wie dies aus Figur 2b hervorgeht, weist diese den Vorteil auf, dass die Feder über einen bestimmten Hub eine konstante Kraft auf die Punktionsstelle ausübt. Sie unterscheidet sich damit von der Kennlinie einer herkömmlichen Feder gemäß Figur 2a, bei der sich die ausgeübte Kraft linear mit der Auslenkung, d.h. dem Hub vergrößert.

Die Konstantkraftfeder bewirkt, dass die Kraft, mit der das Band an die Punktionsstelle gepresst wird, über einen bestimmten Hub konstant ist. Die ideale Position ist in Figur 2b mit dem Bezugszeichen P1 gekennzeichnet. Diese Position stellt die Mitte des Hubes bei konstanter Kraft dar.

Das Bezugszeichen 30 kennzeichnet ein Element in Form einer herkömmlichen Feder, das wie in Figur 1 dargestellt zu der Konstantkraftfeder 10 parallel oder in Serie angeordnet ist. In einer bevorzugten Ausführungsform ist die herkömmliche Feder jedoch nicht vorhanden.

Dieses Element 30 dient dazu, im Fall eines zu starken Anziehens außerhalb der Idealposition P1 oder im Notfall die Punktionsstelle mittels des Verschlusses 40 fester abzudrücken, um eine Blutung stoppen zu können. Das Element 30 kann hierfür z.B. partiell bis zu einem Anschlag über eine konventionelle Federkonstante verfügen (Elastisches Modul) oder als eine Art "Sicherungskette" ausgeführt sein.

Wie aus Figur 1 ersichtlich kann das Band als Kabelbinder oder wie ein Kabelbinder ausgeführt sein. Der Begriff "Band" ist weit auszulegen und umfasst beispielsweise auch Manschetten etc.

Mit dem Bezugszeichen 50 ist eine Skala in Form eines transparenten Fensters (z.B. aus Plastik) gekennzeichnet, das sich vorzugsweise über der Konstantkraftfeder befindet.

Die Skala weist eine max. und min. Markierung auf, die den Bereich der Konstantkraft anzeigen. Sie dient zum idealen Anziehen des Kabelbinders oder eines sonstigen Bandes. Zwischen den max. und min. Markierungen befindet sich der Normalbereich, der in Figur 2b mit P1 markiert ist.

Der Verschluss 40 kann darüber hinaus wieder öffenbar sein (Reuse, Entfernen der Abdrückhilfe).

Verschiedene Stärken der Abdrückhilfe (Konstantkraftfeder) auch unter Berücksichtigung des Unterarmumfangs einer Person/eines Patienten können angeboten werden. Dies kann z.B. durch farbliche Kodierung der "Abdrückhilfe/Armbändchen" kenntlich gemacht werden.

In einer bevorzugten Ausführung sind als Vorteile gegenüber dem Stand der Technik zu nennen:
- Eigensicher, da ein zu festes Abdrücken durch die Konstantkraftfeder konstruktiv ausschließbar ist, insofern nicht das genannte Element vorgesehen wird. Ist dieses vorgesehen, wird dies jedoch dem Bediener durch die Skala deutlich, dass er den Bereich der mechanischen Druckregelung durch das Konstantkraftfederelement verlässt.
- Vorzugsweise ist die Abdrückhilfe wieder manuell über den Verschluss öffenbar.
- Einfach in der Konstruktion und damit preisgünstig. Keine teure und aufwendige Hard- und Softwareentwicklung. Vereinfachung der Risikoanalyse.
- Erhöhter Tragekomfort (durch schmales Design ohne Verkabelung) und mechanische Druckregelung durch Konstantkraftfeder.
- Anschlag zum Festziehen im Notfall bei drohendem Blutverlust möglich.
- Risiko zur Beschädigung des venösen Zugangs sinkt.
- Ausgleich von Muskelbewegungen mit dem Arm/Hand (mechanische Regelung).
- Einfach zu benutzen, d.h. auch im Heimbereich durch wenig geschultes Personal.
- Hohe Verfügbarkeit (kein Akku und keine Ladezustandsabhängigkeit, ...).
- Wiederverwendbar oder als Disposablegeschäft zwecks Hygiene.
- Höheres Vertrauen der Anwender, da Gefahr des zu festen Anziehens entschärft bzw. konstruktiv verhindert wird.
- Leichtere, technische Umsetzung ohne großen Sustaining-Aufwand (Abkündigungen Elektronik HW ...).
- Preisgünstiger realisierbar

## Patentansprüche

1. Abdrückhilfe zum Abdrücken einer Punktionsstelle eines Patienten, wobei die Abdrückhilfe ein umlaufendes Band (10) umfasst, dessen Umfang verstellbar ist, wobei in dem umlaufenden Band (10) eine Konstantkraftfeder (20) angeordnet ist und sich auf der Konstantkraftfeder (20), vorzugsweise auf der Außenseite der Konstantkraftfeder (20), ein Anzeigeelement (50) befindet, das ausgebildet ist, die Auslenkung der Konstantkraftfeder (20) anzuzeigen und dem Nutzer den Bereich der Konstantkraft anzuzeigen.

2. Abdrückhilfe nach Anspruch 1, **dadurch gekennzeichnet, dass** ein Element vorgesehen ist, mittels dessen die ausgeübte Kraft über den Bereich der durch die Konstantkraftfeder (20) aufgebrachten Konstantkraft erhöht werden kann.

3. Abdrückhilfe nach Anspruch 2, **dadurch gekennzeichnet, dass** es sich bei dem Element um eine Feder handelt, die vorzugsweise wenigstens bereichsweise eine lineare Abhängigkeit zwischen Hub und Kraft aufweist.

4. Abdrückhilfe nach Anspruch 2 oder 3, **dadurch gekennzeichnet, dass** das Element zu der Konstantkraftfeder (20) parallel geschaltet ist.

5. Abdrückhilfe nach einem der vorhergehenden Ansprüche, **dadurch gekennzeichnet, dass** das Band (10) zwei Enden aufweist, wobei das eine Ende des Bandes (10) in das andere Ende des Bandes (10) einsteckbar und dort arretierbar ist.

6. Abdrückhilfe nach einem der vorhergehenden Ansprüche, **dadurch gekennzeichnet, dass** das Band (10) einen Verschluss (40) aufweist, der manuell lösbar ist.

7. Abdrückhilfe nach einem der vorhergehenden Ansprüche, **dadurch gekennzeichnet, dass** die Konstantkraftfeder (20) mechanisch und/oder magnetisch ausgeführt ist.

8. Abdrückhilfe nach einem der vorhergehenden Ansprüche, **dadurch gekennzeichnet, dass** die Abdrückhilfe eine farbliche Kodierung aufweist, die mit der durch die Konstantkraftfeder (20) aufgebrachte Kraft korreliert ist.

## Claims

1. Compression aid for compressing a puncture site of a patient, wherein the compression aid comprises a peripheral band (10) whose circumference can be adjusted, wherein a constant force spring (20) is arranged in the peripheral band (10) and an indicator (50) is located on the constant force spring (20), preferably on the outer side of the constant force spring (20), and is configured to indicate the deflection of the constant force spring (20) and to indicate the range of the constant force to the user.

2. Compression aid in accordance with claim 1, **characterized in that** an element is provided by means of which the exerted force can be increased over the range of the constant force applied by the constant force spring (20).

3. Compression aid in accordance with claim 2, **characterized in that** the element is a spring that preferably at least regionally has a linear dependency between the stroke and the force.

4. Compression aid in accordance with claim 2 or claim 3, **characterized in that** the element is connected in parallel with the constant force spring (20).

5. Compression aid in accordance with any one of the preceding claims, **characterized in that** the band (10) comprises two ends, wherein the one end of the band (10) is insertable into the other end of the band (10) and is fixable there.

6. Compression aid in accordance with any one of the preceding claims, **characterized in that** the band (10) comprises a manually releasable closure (40).

7. Compression aid in accordance with any one of the preceding claims, **characterized in that** the constant force spring (20) is configured as mechanical and/or magnetic spring.

8. Compression aid in accordance with any one of the preceding claims, **characterized in that** the compression aid comprises a color coding that is correlated with the force applied by the constant force spring (20).

## Revendications

1. Tourniquet destiné à comprimer un site de ponction d'un patient, dans lequel le tourniquet comprend une bande périphérique (10), dont la périphérie peut être ajustée, dans lequel un ressort à force constante (20) est disposé dans la bande périphérique (10) et un élément d'affichage (50), qui est réalisé pour afficher la déviation du ressort à force constante (20) et pour indiquer à l'utilisateur la zone de la force constante, se trouve sur le ressort à force constante (20), de préférence sur le côté extérieur du ressort à force constante (20).

2. Tourniquet selon la revendication 1, **caractérisé en ce qu'**est prévu un élément, au moyen duquel la force exercée peut être augmentée au-dessus de la plage de la force constante appliquée par le ressort à force constante (20).

3. Tourniquet selon la revendication 2, **caractérisé en ce que** l'élément est un ressort, qui présente de préférence au moins par endroits une dépendance linéaire entre course et force.

4. Tourniquet selon la revendication 2 ou 3, **caractérisé en ce que** l'élément est monté de manière parallèle par rapport au ressort à force constante (20).

5. Tourniquet selon l'une quelconque des revendications précédentes, **caractérisé en ce que** la bande (10) présente deux extrémités, dans lequel une extrémité de la bande (10) peut être enfichée dans l'autre extrémité de la bande (10) et peut y être bloquée.

6. Tourniquet selon l'une quelconque des revendications précédentes, **caractérisé en ce que** la bande (10) présente une fermeture (40), qui peut être déclenchée manuellement.

7. Tourniquet selon l'une quelconque des revendications précédentes, **caractérisé en ce que** le ressort à force constante (20) est réalisé de manière mécanique et/ou magnétique.

8. Tourniquet selon l'une quelconque des revendications précédentes, **caractérisé en ce que** le tourniquet présente un codage couleur, qui est mis en corrélation avec la force appliquée par le ressort à force constante (20).
